# EUROPÄISCHE PATENTSCHRIFT

(11) **EP 0 523 356 B1**
(45) Veröffentlichungstag und Bekanntmachung des Hinweises auf die Patenterteilung: **18.12.1996**
(21) Anmeldenummer: 92108864.7
(22) Anmeldetag: 26.05.1992
(51) Int. Cl.: G01N 33/50

(54) **Burst-Test**
Burst-test
Test de poussée

(30) Priorität: 28.05.1991 DE 4117459
(43) Veröffentlichungstag der Anmeldung: 20.01.1993
(73) Patentinhaber: Orpegen Medizinisch-Molekularbiologische Forschungsgesellschaft m.b.H., 69115 Heidelberg (DE)
(72) Erfinder: Nebe, Karl Thomas, Dr.-Med., W-8400 Regensburg (DE); Hartmann, Karin, W-6800 Mannheim 31 (DE)
(74) Vertreter: Huber, Bernhard, Dipl.-Chem.

(56) Entgegenhaltungen:
- EP-A- 0 435 226
- HIROSHIMA JOURNAL OF MEDICAL SCIENCES vol. 34, no. 1, March 1985, pages 53 - 60; K.TAGA ET AL.: 'Flow Cytometric Assassment of Neutrophil Oxidative Metabolism in Chronic Granulomatous Disease on Small Quantities of Whole Blood : Heterogeneity in Female Patients.'
- NATUR WISSENSCHAFTEN vol. 75, 1988, pages 354 - 355; G.ROTHE ET AL.:Dihydrorhodamine 123: a New Flow Cytometric Indicator for Respiratory Burst Activity in Neutrophil Granulocytes."
- JOURNAL OF IMMUNOLOGICAL METHODS vol. 131, 1990, pages 269 - 275; A. EMMENDOERFFER ET AL.: 'A fast and easy method to determine the production of reactive oxygen intermediates by human and murine phagocytes using dihydrorhodamine 123.'
- JOURNAL OF LEUKOCYTE BIOLOGY vol. 43, no. 4, April 1988, pages 304 - 310; J.P. ROBINSON ET AL.: 'Measurement of Intracellular Fluorescence of Human Monocytes Relative to Oxidative Metabolism.'
- ACTA ENDOCRINOLOGICA vol. 124, no. 5, May 1991, pages 589 - 594; G.L.SPADONI ET AL.: 'Enhancement by growth hormone of phorbol diester-stimulated respiratory burst in human polymorphonuclear leukocytes.'

## Beschreibung

Die vorliegende Erfindung betrifft ein Verfahren zur Bestimmung der intrazellulären oxidativen Vernichtung von Fremdpartikeln in Säugerleukozyten sowie ein dafür geeignetes Reagenzien-Kit.

Der Säugerorganismus verfügt zur Abwehr von eindringenden Mikroorganismen sowie zur Beseitigung von alten und beschädigten Zellen oder Zelltrümmern über Mechanismen, die zur Auflösung dieser unerwünschten Bestandteile führen. Zunächst werden die unerwünschten Bestandteile an der Oberfläche der zur Phagozytose befähigten Leukozyten in peripherem Blut, im wesentlichen Monozyten, Makrophagen und polymorphkernige Leukozyten, gebunden. Wenn diese zur Phagozytose spezialisierte Zelle den gebundenen Bestandteil als "fremd" erkennt, wird der Bestandteil von der Zelle umschlossen und dann durch den sogenannten "oxidativen Burst" vernichtet.

Verminderte Burst- bzw. Phagozytoseleistungen werden u.a. bei angeborenen Defekten, wie der chronischen granulomatösen Erkrankung (CGD), der "adhesive glycoprotein deficiency", dem "hyperimmunoglobulin E-recurrent infection syndrome" (HIE), der Chediak-Higashi disease (CHD), der "specific granule deficiency" und bei der Myeloperoxidase-Defizienz gefunden. Erworbene Defekte der Funktion von neutrophilen Granulozyten sind bei Traumata, Diabetes, Nierenversagen, Alkoholismus und verschiedenen Infektionen zu beobachten (Donabedian, in: Phagocytes and Disease, herausgegeben von Klempner M.S. et al., (1989), Kluwer, Dordrecht).

Für die klinische Diagnostik ist es wichtig, bei Vorliegen einer Immunschwäche festzustellen, inwieweit die Fähigkeit der Leukozyten zum oxidativen Burst beeinträchtigt sind. Zum Nachweis des oxidativen Bursts sind bereits verschiedene Verfahren bekannt, bei denen man in isolierten Leukozyten mit Hilfe von fluoreszierenden Substanzen, die durch Oxidation aktiviert werden, die durch den oxidativen Burst resultierende Fluoreszenz innerhalb der Zelle mißt.

Ryan et al. (J.Immunol.Meth. 130 (1990), 223-233) beschreiben ein Verfahren zur Stimulation von menschlichen Neutrophilen durch Immunkomplexe, um einen oxidativen Burst hervorzurufen und dessen Aktivität zu messen. Die Immunkomplexe bestehen aus Riderserumalbumin, das kovalent mit der nicht-fluoreszierenden Fluoreszenzfarbstoffvorstufe 2',7'-Dichlordihydrofluorescein (DCFH) kovalent verknüpft und zur Bildung eines Immunkomplexes mit Anti-BSA-Immunglobulin behandelt worden sind. Der resultierende Komplex ist ein wirksamer Aktivator zur Stimulation des oxidativen Bursts, wobei das Ausmaß des oxidativen Bursts dem Auftreten der oxidierten Form des Fluoreszenzfarbstoffs 2',7'-Dichlorfluorescin (DCF) entspricht.

Emmendorffer et al. (J.Immunol.Meth. 131 (1990), 269-275) beschreiben ein Verfahren zur Bestimmung des oxidativen Bursts in humanen und murinen Phagozyten unter Verwendung der nicht fluoreszierenden reduzierten Fluoreszenzfarbstoffvorstufe Dihydrorhodamin 123. Als Testmaterial werden Leukozyten verwendet, die durch Zentrifugations- und Lyseschritte von Erythrozyten gereinigt wurden.

Nachteil der oben genannten Verfahren ist, daß sie nicht mit Vollblut durchgeführt wurden und daher aufwendig sind. Weiterhin führt die Aufreinigung der Leukozyten zu Artefakten, Störanfälligkeit und mangelnder Reproduzierbarkeit.

Taga et al. (Hiroshima Journal of Medical Sciences 34, 1, (1985), 53-60) offenbaren ein Verfahren zur Bestimmung des oxidativen Bursts aus Vollblut, wobei eine heparinisierte Blutprobe zunächst mit einer Fluoreszenzsubstrat-Vorstufe, nämlich Dichlorfluorescindiacetat (DCFH-DA) inkubiert wird, danach die Stimulation durch PMA-Zugabe durchgeführt wird und nach Lyse der Erythrozyten mit NH₄Cl die Fluoreszenz bestimmt wird. Dieses Verfahren ist ausreichend, um einen Totalausfall des oxidativen Bursts wie etwa bei CGD (Chronic Granulomatous Disease) zu erfassen. Es wäre jedoch auch wünschenswert über ein Verfahren zu verfügen, mit dem die Erfassung von Krankheitsbildern mit moderaten Veränderungen des oxidativen Bursts möglich ist.

Aufgabe der vorliegenden Erfindung war es somit, ein Verfahren zu entwickeln, bei dem die Nachteile des Standes der Technik mindestens teilweise beseitigt sind. Insbesondere sollte ein Verfahren entwickelt werden, bei dem die Bestimmung des oxidativen Bursts direkt in nicht aufgereinigten Körperflüssigkeiten, insbesondere in Vollblut durchgeführt werden kann.

Die erfindungsgemäße Aufgabe wird gelöst durch ein Verfahren zur Bestimmung der intrazellulären oxidativen Vernichtung von Fremdpartikeln in Säugerleukozyten, welches dadurch gekennzeichnet ist, daß man
(a) eine Vollblutprobe mit einer definierten Menge von mindestens einem Leukozyten-Stimulans versetzt, unter Schütteln 2 bis 120 Minuten bei einer Temperatur von 30 bis 40°C inkubiert,
(b) eine definierte Menge einer Substrat-Lösung zugibt, die eine inaktive Fluoreszenzsubstrat-Vorstufe enthält, die durch Oxidation aktiviert werden kann und im aktiven Zustand vorzugsweise an einen intrazellulären Bestandteil bindet, und erneut unter Schütteln 2 bis 120 Minuten bei einer Temperatur von 30 bis 40°C inkubiert,
(c) ein Lysereagenz zugibt, das in der Probelösung vorhandene Erythrozyten lysiert und
(d) anschließend die Fluoreszenz bestimmt.

Ein wichtiger Punkt des erfindungsgemäßen Verfahrens ist, daß der oxidative Burst in Vollblut gemessen werden kann. Die Phagozytose und der nachfolgende oxidative Burst finden direkt im nicht separierten Vollblut statt und erst anschließend erfolgt Lysis der Erythrozyten und Fixierung der Leukozyten. Dadurch wird eine aufwendige Aufreinigung der Leukozyten vor der Oxidasereaktion vermieden, wodurch die Meßgenauigkeit erhöht wird. Zur Bestimmung des oxidativen Bursts wird vorzugsweise heparinisiertes Vollblut eingesetzt.

Die Erfindung kann mit jeder Art von Säugerleukozyten durchgeführt werden, sofern diese zur intrazellulären oxidativen Vernichtung von Fremdpartikeln, d.h. einem oxidativen Burst in der Lage sind. Vorzugsweise findet die Bestimmung an menschlichen Leukozyten statt.

In einer bevorzugten Ausführungsform des erfindungsgemäßen Verfahrens führt man zwei parallele Bestimmungen mit unterschiedlichen Leukozytenstimulantien durch, von denen eines eine starke oxidative Reaktion und ein anderes eine schwache oxidative Reaktion in der getesteten Zelle hervorruft. Als starke Stimulantien kann man beispielsweise opsonisierte, d.h. mit Antikörpern und Komplement aus gepoolten Seren vorbehandelte Bakterien oder Phorbolester (z.B. Phorbol-12-Myristat-13-Acetat) verwenden. Vorzugsweise verwendet man opsonisierte Bakterien, insbesondere E.coli, Staphylococcus aureus oder Pseudomonas aeruginosa, besonders bevorzugt E.coli-Bakterien. Die Konzentration der opsonisierten Bakterien im Test beträgt vorzugsweise etwa 10⁶ bis 10⁷ Bakterien pro 100 µl Körperflüssigkeit.

Ein Leukozyten-Stimulans, das eine schwache oxidative Reaktion in der Zelle hervorruft, ist beispielsweise ein Komplementfaktor (z.B. Komplementfaktor 5A) oder ein chemotaktisches Peptid mit einem N-terminalen N-Formyl-Rest, insbesondere ein solches, das aus der Gruppe, bestehend aus fMLP (N-Formyl-Met-Leu-Phe) oder Strukturanaloga davon, z.B. N-Formyl-Met-Leu-Phe-Benzylamid, N-Formyl-Met-Leu-Phe-Methylester, N-Formyl-Met-Leu-Phe-Phe, N-Formyl-Met-Phe und N-Formyl-NorLeu-Leu-Phe, ausgewählt ist. Die Endkonzentration des chemotaktischen Peptids in der Probe ist etwa 10⁻⁵ bis 10⁻⁷ mol/l, vorzugsweise 10⁻⁶ mol/l.

Durch das starke Stimulans wird ein maximaler oxidativer Burst in den Leukozyten hervorgerufen ("high control"), während durch das schwache Stimulans nur eine vergleichsweise schwache Reaktion ausgelöst wird ("low control"), bei der man auch additive Effekte von weiteren Prüfsubstanzen, z.B. Immunmodulatoren (Tumor-Nekrosis-Faktor (TNF), Granulozyten-Makrophagen-Koloniestimulierender Faktor (GM-CSF)) bestimmen kann.

Als inaktive Fluoreszenzsubstratvorstufe bzw. als fluorogenes Substrat verwendet man eine Substanz, die durch Oxidation in eine fluoreszierende Substanz umgewandelt werden kann und im aktiven Zustand an einen intrazellulären Bestandteil bindet, so daß eine Diffusion aus der Zelle möglichst gering gehalten wird. Vorzugsweise verwendet man als Fluoreszenzsubstratvorstufe Dihydro- rhodamin 123 (DHR), ein ungeladenes und nichtfluoreszierendes Derivat des Fluoreszenzfarbstoffs Rhodamin 123 (R123), der innerhalb der Zelle an Mitochondrien bindet. Die Herstellung und Verwendung von DHR als Indikator zur Bestimmung des oxidativen Bursts in neutrophilen Granulozyten wurde bereits beschrieben (Rothe et al., Naturwissenschaften 75 (1988), 354-355).

Es können jedoch auch andere Farbstoffe verwendet werden, z.B. Fluoresceinderivate wie etwa Dichlorfluorescindiacetat, welches durch intrazelluläre Esterasen zu Dichlorfluorescin umgewandelt wird und dann zu Dichlorfluorescein oxidiert wird. Die letztgenannte Farbstoff-Vorstufe muß jedoch an einen Immunkomplex gekoppelt werden, um in der Zelle gehalten zu werden und ist daher für das erfindungsgemäße Verfahren im allgemeinen weniger bevorzugt.

Alle Oxidasesubstrate sind naturgemäß sehr leicht durch Luftsauerstoff oxidierbar und werden daher vorzugsweise unter inertem Gas (z.B. Argon) gasdicht ampulliert. Vorzugsweise wird das erfindungsgemäße Verfahren durchgeführt, indem das Substrat auf Filterscheiben geträufelt wird, um ein reproduzierbares Auflösen und Verdünnen zu ermöglichen.

Die Endkonzentration des Substrats kann z.B. etwa 0,05 bis 1 µg, vorzugsweise 0,2 µg auf 100 µl getestete Körperflüssigkeit betragen. Die Einstellung dieser Endkonzentration erfolgt beispielsweise mit Hilfe der Filterscheiben. Dazu werden 10 µl Dihydrorhodamin 123 Lösung (1 mg/ml in DMF) auf eine Filterscheibe gegeben. Für den Versuch erfolgt dann ein Verdünnungsschritt von 1:100. Von dieser verdünnten Lösung (10 µg/ml) werden wiederum 20 µl auf 100 µl Testlösung eingesetzt. Daraus resultiert die besonders bevorzugte Substratkonzentration von 0,2 µg Substrat auf 100 µl Testflüssigkeit. Konzentrationen, die oberhalb von 1 µg Substrat pro 100 µl Testflüssigkeit liegen, führen (bei Verwendung von DMF als Lösungsmittel) zu hohen Konzentrationen an organischem Lösungsmittel, die für Zellen toxisch sind. Zu niedere Mengen an Substrat (< 0,05 µg/100 µl Testflüssigkeit) sind wiederum aufgrund der resultierenden schwächeren Fluoreszenzintensität im Test weniger bevorzugt.

Die Inkubationszeit der Leukozyten mit dem Stimulans (Schritt (a) des erfindungsgemäßen Verfahrens) kann 2 bis 120 Minuten, vorzugsweise 5 bis 20 Minuten, besonders bevorzugt etwa 10 Minuten bei einer Temperatur von 30 bis 40°C betragen. Die Inkubationszeit der Zellen mit der Substratlösung (Schritt (b) des erfindungsgemäßen Verfahrens) kann ebenfalls 2 bis 120 Minuten, vorzugsweise 5 bis 20 Minuten, bevorzugt etwa 10 Minuten bei einer Temperatur von 30 bis 40°C betragen. Es ist aber auch möglich, das erfindungsgemäße Verfahren in mehreren parallelen Ansätzen mit verschiedenen Inkubationszeiten durchzuführen, so daß eine Zeitkinetik gemessen werden kann, die genauere Aussagen über den oxidativen Burst zuläßt.

Die Abfolge der Schritte (a) und (b) bei dem erfindungsgemäßen Verfahren ist vorzugsweise so, daß Schritt (b) nach Schritt (a) erfolgt. Es ist jedoch auch möglich, daß die Schritte (a) und (b) gleichzeitig durchgeführt werden.

Zur Lysebehandlung wird dem Reaktionsgemisch ein Lysereagenz zugegeben, das die in der Lösung vorhandenen Erythrozyten zerstört, die nachzuweisenden Leukozyten jedoch praktisch unversehrt läßt. Hierzu wird vorzugsweise eine isotone oder hypotone Salzlösung verwendet, z.B. eine Lösung, die einen für Erythrozyten toxisch hohen Anteil an NH₄Cl sowie gegebenenfalls EDTA enthält. Besonders bevorzugt zur Zerstörung der Erythrozyten sind Lösungen, die Formaldehyd und/oder Diethylenglykol enthalten. Besonders bevorzugt wird eine Lösung verwendet, die als FACS-Lysing Solution (Fa. Becton Dickinson) im Handel erhältlich ist.

Bei dem erfindungsgemäßen Verfahren ist es vorteilhaft, nach der Lysis der Erythrozyten eine DNA-Färbelösung zuzugeben, die einen fluoreszierenden DNA-Farbstoff enthält. Auf diese Weise ist es möglich, Bakterienaggregate von Leukozyten deutlich zu unterscheiden. Als DNA-Farbstoff kann ein hierfür bekannter und geeeigneter Farbstoff verwendet werden, der eine andere Wellenlänge hat als der für die Fluoreszenzmarkierung der Bakterien verwendete Farbstoff. Vorzugsweise verwendet man Propidiumjodid als DNA-Farbstoff. Bei der Auswertung der Fluoreszenz werden dann nur solche Zellen erfaßt, deren DNA-Gehalt mindestens der einer menschlichen Zelle ist, was Bakterienaggregate, Zellbruchstücke oder Aggregationsartefakte ausschließt.

Nach der Lysis der Erythrozyten, die günstigerweise durch 20-minütige Inkubation des Reaktionsgemisches bei Raumtemperatur mit dem Lysereagenz stattfindet, wird zentrifugiert und der Überstand (mit den lysierten Erythrozyten) verworfen. Anschließend werden die Proben mit Waschlösung gewaschen, zentrifugiert und der Überstand abgekippt.

Der letzte Schritt des erfindungsgemäßen Verfahrens ist die Bestimmung der Fluoreszenz. Die Auswertung kann dabei in einem Durchflußzytometer, einem Bildanalysesystem (auf Mikroskopbasis) oder in einem Spektralfluorimeter (auch für Mikrotiterplatten) durchgeführt werden. Vorzugsweise erfolgt die Auswertung in einem Durchflußzytometer, das auf Einzelzellebene durchgeführt wird, so daß eine Unterscheidung von Subpopulation der weißen Blutzellen durch eine geeignete Wahl des Versuchsdesigns möglich ist.

Weiterhin ein Gegenstand der vorliegenden Erfindung ist die Verwendung eines Reagenzien-Kit in einem Verfahren zur Bestimmung der intrazellulären oxidativen Vernichtung von Fremdpartikeln in Säugerleukozyten, wobei das Kit Lösungen von mindestens einem Stimulans für Säugerleukozyten, eine Substratlösung mit einer inaktiven Fluoreszenzsubstratvorstufe, ein Lysereagenz sowie übliche Puffer physikalisch getrennt voneinander enthält. Die "Lösungen" des Reagenzien-Kits können sowohl fertige Lösungen sein oder auch Lyophilisate darstellen, aus denen der Anwender die Lösungen erst herstellt.

Das Reagenzien-Kit enthält vorzugsweise Lösungen mit unterschiedlichen Stimulantien, von denen das eine eine starke und das andere eine schwache oxidative Reaktion in der Zelle hervorruft. Weiterhin ist bevorzugt, daß das Reagenzien-Kit zusätzlich eine Färbelösung mit einem fluoreszierenden DNA-Farbstoff enthält, der eine andere Fluoreszenzwellenlänge als die des aktivierten Fluoreszenzsubstrats besitzt. Wird als Fluoreszenzsubstrat Rhodamin 123 verwendet, so eignet sich als fluoreszierender DNA-Farbstoff beispielsweise Propidiumjodid. Ferner können die Reagenzienlösungen des Kits auch ein Konservierungsmittel, insbesondere Thimerosal enthalten.

In einer besonders bevorzugten Ausführungsform enthält das Reagenzien-Kit die folgenden Lösungen:
1. stabilisierte opsonisierte E.coli-Suspension mit Konservierungsmittel,
2. Lösung des chemotaktischen Peptids N-Formyl-Met-Leu-Phe in einem organischen, flüchtigen Lösungsmittel,
3. gasdichte Ampullen (unter Inertgasatmosphäre) der Fluoreszenzsubstratvorstufe Dihydrorhodamin 123 mit einer Substratscheibe (durch Injektion von Waschlösung direkt vor Gebrauch zu rekonstituieren, danach verwerfen),
4. Salze für die Waschlösung zur Rekonstitution in jeweils 100 ml H₂O bidest., vor Gebrauch 1:10 mit H₂O bidest. verdünnen (ergibt jeweils 1 l gebrauchsfertige 1x-Waschlösung), enthält Konservierungsmittel,
5. Lösung des Lysereagenz zur Lysis der Erythrozyten und simultaner Fixierung der Leukozyten und
6. DNA-Färbelösung zur Ausgrenzung der Bakterien bei der Leukozytenanalyse, enthält Konservierungsmittel.

Als Meßgerät wird vorzugsweise ein Durchflußzytometer (z.B. FACScan, Firma Becton Dickinson) mit 488 nm Lichtanregung (Argonlaser oder Quecksilberdampflampe) verwendet.

Der Reagenzien-Kit wird günstigerweise bei +4°C im Dunklen aufbewahrt. Die Bakterien müssen vor der Verwendung gründlich gemixt oder mit einer Spritze vereinzelt werden. Die Haltbarkeit eines derartigen unbenutzten Kits beträgt mindestens 3 Monate. Die Substratlösung und die N-Formyl-Met-Leu-Phe-Gebrauchslösung müssen nach der sofortigen Verwendung verworfen werden.

Die Erfindung wird weiterhin durch die folgenden Beispiele veranschaulicht.

### Beispiel 1

### Bestimmung der Phagozytoseaktivität von Monozyten und Granulozyten in Vollblut

Frisch entnommenes, heparinisiertes Vollblut wird gemixt (Vortex) und auf dem Boden eines 5 ml-Röhrchens aliquotiert, wobei pro Ansatz 100 µl verwendet werden. Durch EDTA oder Citrat stabilisiertes Vollblut ist für das Verfahren nicht sonderlich geeignet. Die Blutproben werden vor der Zugabe der Bakterien ca. 15 Minuten im Eiswasserbad inkubiert, um auf 0°C abgekühlt zu sein.

Die vorgekühlten E.coli-Bakterien (1 x 10⁹ pro ml) werden gut gemixt (Vortex) und 20 µl pro Ansatz zum Vollblut pipettiert ("high control", Röhrchen 1). Gegebenenfalls können 20 µl N-Formyl-Met-Leu-Phe-Gebrauchslösung als "low control" in ein weiteres Röhrchen (Röhrchen 2) gegeben werden (Endkonzentration 10⁻⁶ mol/l). 20 µl Waschlösung (phosphatgepufferte Salzlösung ohne Calcium²⁺ und Magnesium²⁺ mit 0,02 % EDTA) werden als Negativkontrolle in ein drittes Röhrchen (Röhrchen 3) gegeben.

Alle Röhrchen werden nochmals gemixt (Vortex) und 10 Minuten lang bei 37°C im Wasserbad unter starkem Schütteln inkubiert (bei einer kinetischen Messung können auch längere Inkubationszeiten erforderlich sein).

Nach 10 Minuten erfolgt die Zugabe von 20 µl frisch angesetzter Substratlösung und Mischen (Vortex) (Endkonzentration 0,2 µg Dihydrorhodamin 123 auf 100 µl Körperflüssigkeit). Alle Röhrchen werden 10 Minuten bei 37°C im Wasserbad unter starkem Schütteln inkubiert (bei kinetischen Messungen kann auch hier eine längere Inkubation erfolgen).

Am Ende der Inkubationszeit werden alle Proben zum Stoppen der Phagozytose dem 37°C-Wasserbad entnommen. Das Vollblut wird anschließend mit 2 ml Lyse-Lösung (1x FACS Lysing Solution, Firma Becton Dickinson, Best.Nr. 920002) bei Raumtemperatur aufgefüllt und gründlich gemischt (Vortex). Das Blut wird durch 20-minütige Inkubation bei Raumtemperatur lysiert und fixiert. Anschließend wird 5 Minuten bei 250 g und 4°C in einer Kühlzentrifuge zentrifugiert und der Überstand abgekippt.

Dann werden die Proben 1x mit 3 ml kalter Waschlösung gewaschen und 5 Minuten lang bei 250 g und 4°C in einer Kühlzentrifuge zentrifugiert. Der Überstand wird abgekippt.

Zur DNA-Färbung werden 100 µl DNA-Färbelösung (0,04 mg/ml Propidiumjodid (Sigma) in PBS) zugegeben, gemischt und 10 Minuten bei 0°C in einem lichtgeschützten Eiswasserbad inkubiert. Die Messung muß innerhalb von 30 Minuten erfolgen. Das Volumen der Zellprobe kann durch Zugabe von Waschlösung erhöht werden.

Die Auswertung erfolgt im Durchflußzytometer (FACScan) unter Verwendung von handelsüblicher Software (z.B. Consort30). Dazu werden mindestens 15.000 Leukozyten bei blaugrüner Lichtanregung (488 nm beim Argonlaser) im Durchflußcytometer aquiriert. Während der Datenaufnahme wird ein sogenanntes Live Gate im Histogramm der Rotfluoreszenz (Propidiumjodid) auf diejenigen Ereignisse gesetzt, die mindestens den DNA-Gehalt einer menschlichen Zelle besitzen (= Ausschluß von Bakterienaggregaten, die die gleichen Streulichteigenschaften wie Leukozyten besitzen). Dieses Gating spart Speicherkapazität, kann aber auch nach der Datenaufnahme erfolgen.

Ausgewertet werden der prozentuale Anteil der oxidierenden Zellen (Rekrutierung) sowie deren mittlere Fluoreszenzintensität (Menge an umgesetztem Substrat, Aktivierung). Dadurch wird das entsprechende Leukozytencluster im Software-Programm im Streulichtdiagramm (lin SSC/y gegen lin FSC/x) gegatet und dessen Histogramm der Grünfluoreszenz (FL1) analysiert (SSC = Seitwärtsstreulicht (side scatter); FSC = Vorwärtsstreulicht (forward scatter); lin = lineare Verstärkung)

Dazu wird im Grünfluoreszenzhistogramm eine Region auf die gegenüber der Negativkontrolle positiven, grünfluoreszierenden Zellen gesetzt (= Anteil der oxidierenden Zellen) und nur deren mittlere Fluoreszenzintensität (Mean) abgelesen (= mittlere Aktivität pro einzelnem Leukozyt). Das Streulichtfenster für Granulozyten ist so zu bemessen, daß auch die im höchsten SSC-Kanal liegenden Zellen miterfaßt werden, oder es muß der SSC-Verstärkungsfaktor reduziert werden.

| Erwartungswerte: | | |
|---|---|---|
| bezogen auf: (10 + 10 Minuten Inkubation) | % oxidierende Granulozyten | mittlere Fluoreszenzintensität (4 Dekaden, 1024 Kanäle) |
| Bakterien | 70-95 | 80-300 |
| N-Formyl-Met-Leu-Phe | 1-5 | 10-100 |

## Patentansprüche

1. Verfahren zur Bestimmung der intrazellulären oxidativen Vernichtung von Fremdpartikeln in Säugerleukozyten,
**dadurch gekennzeichnet**,
daß man
(a) eine Vollblutprobe mit einer definierten Menge von mindestens einem Leukozyten-Stimulans versetzt, unter Schütteln 2 bis 120 Minuten bei einer Temperatur von 30 bis 40°C inkubiert,
(b) eine definierte Menge einer Substrat-Lösung zugibt, die eine inaktive Fluoreszenzsubstrat-Vorstufe enthält, die durch Oxidation aktiviert werden kann und im aktiven Zustand vorzugsweise an einen intrazellulären Bestandteil bindet, und erneut unter Schütteln 2 bis 120 Minuten bei einer Temperatur von 30 bis 40°C inkubiert,
(c) ein Lysereagenz zugibt, das in der Probelösung vorhandene Erythrozyten lysiert und
(d) anschließend die Fluoreszenz bestimmt.

2. Verfahren nach Anspruch 1,
**dadurch gekennzeichnet**,
daß man zwei parallele Bestimmungen mit unterschiedlichen Leukozyten-Stimulantien durchführt, von denen das eine starke und das andere eine schwache oxidative Reaktion in der Zelle hervorruft.

3. Verfahren nach Anspruch 2,
**dadurch gekennzeichnet**,
daß man als starkes Leukozyten-Stimulans opsonisierte Bakterien oder Phorbolester verwendet.

4. Verfahren nach Anspruch 3,
**dadurch gekennzeichnet**,
daß man als opsonisierte Bakterien E.coli, Staphylococcus aureus oder Pseudomonas aeruginosa verwendet.

5. Verfahren nach Anspruch 2
**dadurch gekennzeichnet**,
daß man als schwaches Leukozyten-Stimulans ein chemotaktisches Peptid oder Peptidderivat mit N-terminalem Formyl-Met-Rest, insbesondere N-Formyl-Met-Leu-Phe verwendet.

6. Verfahren nach einem der vorhergehenden Ansprüche,
**dadurch gekennzeichnet**,
daß man die Bestimmung der Leukozytenaktivität in Gegenwart von mindestens einer weiteren Prüfsubstanz durchführt.

7. Verfahren nach Anspruch 6,
**dadurch gekennzeichnet**,
daß man als weitere Prüfsubstanz einen körpereigenen Immunmodulator, insbesondere Tumornekrosis-Faktor, Granulozyten-Kolonienstimulierenden Faktor (G-CSF) oder Granulozyten-Makrophagen-Kolonienstimulierenden Faktor (GM-CSF) verwendet.

8. Verfahren nach einem der vorhergehenden Ansprüche,
**dadurch gekennzeichnet**,
daß man als inaktive Fluoreszenzsubstrat-Vorstufe Dihydrorhodamin 123 verwendet.

9. Verfahren nach einem der vorhergehenden Ansprüche,
**dadurch gekennzeichnet**,
daß man die Substratlösung auf eine Filterscheibe gibt, um eine reproduzierbare Auflösung und Verdünnung zu ermöglichen.

10. Verfahren nach einem der vorhergehenden Ansprüche,
**dadurch gekennzeichnet**,
daß man eine heparinisierte Vollblutprobe verwendet.

11. Verfahren nach einem der vorhergehenden Ansprüche,
**dadurch gekennzeichnet**,
daß die Inkubationszeit in Schritt (a) 5 bis 20 Minuten beträgt.

12. Verfahren nach einem der vorhergehenden Ansprüche,
**dadurch gekennzeichnet**,
daß die Inkubationszeit in Schritt (b) 5 bis 20 Minuten beträgt.

13. Verfahren nach einem der vorhergehenden Ansprüche,
**dadurch gekennzeichnet**,
daß man ein Lysereagenz verwendet, das Formaldehyd oder/und Diethylenglykol enthält.

14. Verfahren nach einem der vorhergehenden Ansprüche,
**dadurch gekennzeichnet**,
daß man ein Lysereagenz verwendet, das Ammoniumchlorid und EDTA enthält.

15. Verfahren nach einem der vorhergehenden Ansprüche,
**dadurch gekennzeichnet**,
daß man nach Schritt (c) einen fluoreszierenden DNA-Farbstoff zugibt.

16. Verfahren nach Anspruch 15,
**dadurch gekennzeichnet**,
daß man als fluoreszierenden DNA-Farbstoff Propidiumjodid verwendet.

17. Verfahren nach einem der vorhergehenden Ansprüche,
**dadurch gekennzeichnet**,
daß man Reagenzlösungen verwendet, die ein Konservierungsmittel, insbesondere Thimerosal enthalten.

18. Verwendung eines Reagenzien-Kit in einem Verfahren zur Bestimmung der intrazellulären oxidativen Vernichtung von Fremdpartikeln in Säugerleukozyten nach Anspruch 1,
**dadurch gekennzeichnet**,
daß das Kit Lösungen von mindestens einem Stimulans für Säugerleukozyten, eine Substratlösung mit einer inaktiven Fluoreszenzsubstrat-Vorstufe, ein Lysereagenz sowie übliche Puffer physikalisch getrennt voneinander enthält.

19. Verwendung nach Anspruch 18,
**dadurch gekennzeichnet**,
daß das Reagenzien-Kit ein Lyse- und Fixierungsreagenz enthält.

20. Verwendung nach Anspruch 18 oder 19,
**dadurch gekennzeichnet**,
daß sich die Substratlösung der inaktiven Fluoreszenzsubstrat-Vorstufe unter einer Inertgasatmosphäre befindet.

21. Verwendung nach einem der Ansprüche 18 bis 20,
**dadurch gekennzeichnet**,
daß das Reagenzien-Kit Lösungen mit unterschiedlichen Stimulantien enthält, von denen das eine eine starke und das andere eine schwache oxidative Reaktion in der Zelle hervorruft.

22. Verwendung nach einem der Ansprüche 18 bis 21,
**dadurch gekennzeichnet**,
daß das Reagenzien-Kit weiterhin eine DNA-Färbelösung enthält.

23. Verwendung nach einem der Ansprüche 18 bis 22,
**dadurch gekennzeichnet**,
daß die Reagenzienlösungen ein Konservierungsmittel, insbesondere Thimerosal enthalten.

## Claims

1. Process for the determination of intracellular oxidative destruction of foreign particles in mammalian leucocytes **characterised in that**:
(a) a sample of whole blood is treated with a defined quantity of at least one leucocyte stimulant and incubated at 30-40°C with shaking for 2-120 minutes,
(b) a defined quantity of a substrate solution is added containing an inactive fluorescence substrate precursor which can be activated by oxidation and, in the active state, preferably bound to an intracellular constituent, and incubated again at 30-40°C with shaking for 2-120 minutes,
(c) a lysis reagent is added which lyses any erythrocytes present in the test solution, and
(d) finally the fluorescence is determined.

2. Process according to Claim 1, **characterised by** carrying out two parallel determinations with different leucocyte stimulants, one of which produces a strongly oxidative reaction in the cell and the other a weak one.

3. Process according to Claim 2, **characterised in that** opsonised bacteria or phorbol esters are used as strong leucocyte stimulants.

4. Process according to Claim 3, **characterised in that** E.coli, Staphylococcus aureus or Pseudomonas aeruginosa are used as the opsonised bacteria.

5. Process according to Claim 2, **characterised in that** a chemotactic peptide or peptide derivative with an N-terminal formyl-met-residue, especially N-formyl-met-leu-phe, is used as a weak leucocyte stimulant.

6. Process according to one of the preceding claims, **characterised in that** determination of leucocyte activity is carried out in the presence of at least one additional test substance.

7. Process according to Claim 6, **characterised in that** there is employed as an additional test substance an assimilated immunoregulator, in particular Tumour Necrosis Factor, Granulocyte Colony Stimulating Factor (G-CSF) or Granulocyte Macrophage Colony Stimulating Factor (GM-CSF).

8. Process according to one of the preceding claims, **characterised in that** dihydrorhodamine 123 is used as the inactive fluorescent substrate precursor.

9. Process according to one of the preceding claims, **characterised in that** the substrate solution is introduced onto a filter disc in order to facilitate reproducible solution and dilution.

10. Process according to one of the preceding claims, **characterised in that** a heparinised sample of whole blood is used.

11. Process according to one of the preceding claims, **characterised in that** the incubation time in step (a) is 5 to 20 minutes.

12. Process according to one of the preceding claims, **characterised in that** the incubation time in step (b) is 5 - 20 minutes.

13. Process according to one of the preceding claims, **characterised in that** formaldehyde and/or diethylene glycol are present in the lysing reagent.

14. Process according to one of the preceding claims, **characterised in that** ammonium chloride and EDTA are present in the lysing reagent.

15. Process according to one of the preceding claims, **characterised in that** a fluorescent DNA dye is added after step (c).

16. Process according to Claim 15, **characterised in that** propidium iodide is employed as the fluorescent DNA dye.

17. Process according to one of the preceding claims, **characterised in that** the reagent solutions employed contain a stabilising substance, in particular Thimerosal.

18. Use of a Reagent Kit in a process for the determination of the intracellular oxidative destruction of foreign particles in mammalian leucocytes according to Claim 1, **characterised in that** the kit contains, physically separated from each other, at least one stimulant for mammalian leucocytes, a substrate solution with an inactive fluorescent substrate precursor, a lysis reagent and conventional buffers.

19. Use according to Claim 18, **characterised in that** the Reagent Kit contains a lysis and fixing agent.

20. Use according to Claim 18 or 19, **characterised in that** the substrate solution of the inactive fluorescent substrate precursor is kept under an inert gas atmosphere.

21. Use according to one of Claims 18 to 20, **characterised in that** the Reagent Kit contains solutions of differing stimulants, one of which gives a strong, and the other a weak, oxidative reaction in the cell.

22. Use according to one of Claims 18 to 21, **characterised in that** the Reagent Kit additionally contains a DNA dye solution.

23. Use according to one of Claims 18 to 22, **characterised in that** the reagent solutions contain a preservative, especially Thimerosal.

## Revendications

1. Procédé de détermination de la destruction intracellulaire des particules étrangères par oxydation dans les leucocytes de mammifères, caractérisé en ce que
(a) on ajoute à un échantillon de sang total une quantité définie d'au moins un stimulant des leucocytes, on incube pendant 2 à 120 minutes à une température de 30 à 40°C sous agitation,
(b) on ajoute une quantité définie d'une solution de substrat qui contient un précurseur inactif de substrat fluorescent qui peut être activé par oxydation et qui, à l'état activé, se lie de préférence à un constituant intracellulaire, et on incube de nouveau pendant 2 à 120 minutes à une température de 30 à 40°C sous agitation,
(c) on ajoute un réactif de lyse qui lyse les érythrocytes présents dans la solution d'échantillon et
(d) on détermine ensuite la fluorescence.

2. Procédé selon la revendication 1, caractérisé en ce que l'on réalise deux déterminations parallèles avec des stimulants des leucocytes différents parmi lesquels l'un provoque une forte réaction d'oxydation dans la cellule et l'autre provoque une faible réaction d'oxydation dans la cellule.

3. Procédé selon la revendication 2, caractérisé en ce que l'on utilise comme stimulant fort des leucocytes des bactéries opsonisées ou des esters de phorbol.

4. Procédé selon la revendication 3, caractérisé en ce que l'on utilise comme bactéries opsonisées E. coli, Staphylococcus aureus ou Pseudomonas aeruginosa.

5. Procédé selon la revendication 2, caractérisé en ce que l'on utilise comme stimulant faible des leucocytes un peptide ou dérivé peptidique chimiotactique à reste formyl-Met N-terminal, en particulier N-formyl-Met-Leu-Phe.

6. Procédé selon l'une des revendications précédentes, caractérisé en ce que l'on accomplit la détermination de l'activité des leucocytes en présence d'au moins une autre substance à étudier.

7. Procédé selon la revendication 6, caractérisé en ce que l'on utilise comme autre substance à étudier un immunomodulateur propre de l'organisme, en particulier le facteur nécrosant les tumeurs, le facteur stimulant les colonies de granulocytes (G-CSF) ou le facteur stimulant les colonies de granulocytes et de macrophages (GM-CSF).

8. Procédé selon l'une des revendications précédentes, caractérisé en ce que l'on utilise la dihydrorhodamine 123 comme précurseur inactif de substrat fluorescent.

9. Procédé selon l'une des revendications précédentes, caractérisé en ce que l'on verse la solution de substrat sur un disque filtrant pour permettre une dissolution et une dilution reproductibles.

10. Procédé selon l'une des revendications précédentes, caractérisé en ce que l'on utilise un échantillon de sang total hépariné.

11. Procédé selon l'une des revendications précédentes, caractérisé en ce que le temps d'incubation de l'étape (a) est de 5 à 20 minutes.

12. Procédé selon l'une des revendications précédentes, caractérisé en ce que le temps d'incubation de l'étape (b) est de 5 à 20 minutes.

13. Procédé selon l'une des revendications précédentes, caractérisé en ce que l'on utilise un réactif de lyse qui contient du formaldéhyde et/ou du diéthylèneglycol.

14. Procédé selon l'une des revendications précédentes, caractérisé en ce que l'on utilise un réactif de lyse qui contient du chlorure d'ammonium et de l'EDTA.

15. Procédé selon l'une des revendications précédentes, caractérisé en ce que l'on ajoute un colorant fluorescent de l'ADN après l'étape (c).

16. Procédé selon la revendication 15, caractérisé en ce que l'on utilise l'iodure de propidium comme colorant fluorescent de l'ADN.

17. Procédé selon l'une des revendications précédentes, caractérisé en ce que l'on utilise des solutions de réactifs qui contiennent un conservateur, en particulier le thimérosal.

18. Utilisation d'une trousse de réactifs dans un procédé de détermination de la destruction intracellulaire des particules étrangères par oxydation dans les leucocytes de mammifères selon la revendication 1, caractérisée en ce que la trousse contient, séparés physiquement les uns des autres, des solutions d'au moins un stimulant des leucocytes de mammifères, une solution de substrat contenant un précurseur inactif de substrat fluorescent, un réactif de lyse et des tampons habituels.

19. Utilisation selon la revendication 18, caractérisée en ce que la trousse de réactifs contient un réactif de lyse et de fixation.

20. Utilisation selon la revendication 18 ou 19, caractérisée en ce que la solution de substrat du précurseur inactif de substrat fluorescent se trouve dans une atmosphère de gaz inerte.

21. Utilisation selon l'une des revendications 18 à 20, caractérisée en ce que la trousse de réactifs contient des solutions contenant différents stimulants parmi lesquels l'un provoque une forte réaction d'oxydation dans la cellule et l'autre provoque une faible réaction d'oxydation dans la cellule.

22. Utilisation selon l'une des revendications 18 à 21, caractérisée en ce que la trousse de réactifs contient en outre une solution de colorant de l'ADN.

23. Utilisation selon l'une des revendications 18 à 22, caractérisée en ce que les solutions de réactifs contiennent un conservateur, en particulier le thimérosal.
